# EUROPEAN PATENT APPLICATION

(11) **EP 0 608 040 A2**
(43) Date of publication of application: **27.07.1994**
(21) Application number: 94200143.9
(22) Date of filing: 21.01.1994
(51) Int. Cl.: C08B 30/20, C12S 3/12

(54) **A method for fractionating starch**

(30) Priority: 22.01.1993 NL 9300131
(71) Applicant: Coöperatieve Verkoop- en Productievereniging van Aardappelmeel en Derivaten 'AVEBE' B.A., NL-9641 JA Veendam (NL)
(72) Inventor: Gotlieb, Kornelis Fester, NL-9643 LC Veendam (NL); Bergsma, Jacob, NL-9751 BE Haren (NL); Voogd, Roeland Huibert, NL-9607 PV Foxhol (NL)
(74) Representative: Smulders, Theodorus A.H.J., Ir.

(57) **Abstract**

It has been found that an aqueous starch solution which further contains dissolved phosphate can be fractionated by means of the enzyme potato phosphorylase. Thus, an amylose-rich solid fraction is obtained which can be separated from the reaction mixture, whereafter an amylopectin solution remains, which can be further processed or from which amylopectin can be recovered.

## Description

This invention relates to a method for fractionating starch, in which use is made of the enzyme potato phosphorylase.

Most types of starch consist of granules in which two types of glucose polymers occur, viz. amylose (15-35 wt.% based on the solids) and amylopectin (65-85 wt.% based on the solids). Amylose consists of non-branched or little-branched molecules having an average degree of polymerization of 1,000 to 5,000 (depending on the type of starch). Amylopectin consists of highly branched molecules having an average degree of polymerization of 2,000,000. Amylose and amylopectin possess essentially different properties when used in aqueous solutions and in dried film layers. Amylose has good gelling and film forming properties. Amylopectin, on the other hand, has good properties as thickener, stabilizer and binder. The above-mentioned properties of amylose and amylopectin are only partly operative in solutions and starch films because in them amylose and amylopectin are present in mixed form. In these solutions and films, amylose and amylopectin more or less counteract each other, so that their good properties as separate fractions are partly lost.

Since 1940 many methods have been developed for separating amylose-containing starches into the fractions amylose and amylopectin. These methods are designated by the term fractionation. Through fractionation of starch, enriched amylose and amylopectin fractions are obtained, which possess the more specific properties which are desired in many applications.

Known methods for fractionating starch include chromatographic separation, leaching amylose from gelatinized starch granules, fractionation with complex formers, fractionated precipitation, fractionation by salting out with magnesium sulfate and fractionation by retrogradation. These methods are described by A.H. Young in the chapter "Fractionation of starch" in the book by R.L. Whistler, J.N. Bemiller and
E.F. Pascall (Eds.), Starch: Chemistry and Technology, Second Edition, 1984, Academic Press, pp. 249-283. However, none of these methods proved in the end to be economically feasible.

Because of the specific properties of the fractions amylose and amylopectin obtained from starch through fractionation, there continues to be a need for new methods for fractionating starch. The invention relates to a new method for fractionating starch, in which potato phosphorylase is allowed to act on an aqueous starch solution which also contains dissolved phosphate and the solid amylose-rich fraction obtained is separated from the residual liquid fraction of solution. The invention is based on the surprising phenomenon that in an aqueous starch solution in which potato phosphorylase and phosphate are present, a properly separable solid amylose-rich product can be formed.

By the term starch as used herein is meant a starch having more than 15 wt.% amylose based on the solids. According to the invention, any variety of amylose-containing starch can be used, such as potato starch, maize starch, tapioca starch, wheat starch, sago starch, pea starch and high-amylose starch. Preferably, potato starch is used. Potato starch contains amylose having a relatively high molecular weight. High-molecular amylose gives stronger and more coherent films and coatings. According to the invention, it is also possible to use modified starch, obtained through chemical enzymatic and/or physical modification of native starch. The term starch as used herein also encompasses such modified starches.

The method according to the invention is carried out in an aqueous environment. The starch solution to be used can be prepared by heating an aqueous starch suspension to above the gelatinization temperature of the starch, for instance using a steam jet device (typically designated as jet cooker). The starch concentration to be used is preferably between 3 and 40 wt.% and preferably between 5 and 30 wt.%.

Potato phosphorylase (EC 2.4.1.1.) occurs in potato juice. Potato juice is a product which is released in the industrial production of potato starch from potatoes. The potato juice further contains *inter alia* dissolved proteins, peptides, amides, amino acids, lipids, citric acid, potassium and phosphate salts. According to the invention, this potato juice can in principle be used as source of potato phosphorylase and phosphate. Preferably, however, this potato juice is heated and/or acidified in such a manner that the dissolved proteins coagulate, without the potato phosphorylase being inactivated. Then the potato juice is clarified, the coagulated protein material and other solid components being removed from the solution by filtration or centrifugation. The thus clarified potato juice is a relatively inexpensive source of potato phosphorylase. If so desired, potato phosphorylase can be isolated from this clarified potato juice through known techniques and be used as purified preparation. The potato phosphorylase preparations to be used should only contain minor amounts of objectionable enzymes such as alpha-amylase, phosphatase and/or branching enzyme.

In the practice of the method according to the invention it is necessary that the starch solution also contains a dissolved phosphate compound. As phosphate compound any water-soluble inorganic phosphate compound or glucose-1-phosphate can be used. Potato juice by nature contains both potato phosphorylase and dissolved phosphate compounds and can therefore serve as source for both products. If necessary, additional phosphate can be added to the reaction medium if too little phosphate is present in the potato juice. It is also possible to add purified phosphorylase preparations and phosphate compounds to the starch solution as separate components. The amount of phosphate to be used can be determined by a skilled worker through simple experiments.

The method according to the invention is preferably carried out at pH values between 5 and 9 and preferably between 6 and 8. To keep the pH of the reaction mixture at the desired pH, if necessary a buffer can be used, such as acetates, phosphates, or citrates. The reaction temperature to be used is preferably between 0 and 60°C and preferably between 20 and 45°C. The reaction time is not limited but is preferably between 1 and 100 hours. The reaction is preferably carried out under sterile conditions and in that case in principle no preservative against microbiological decay of the starch solution needs to be used. If necessary, however, preservatives allowed by the relevant official rules can be incorporated into the starch solution to be treated.

In the method according to the invention, the reaction mixture may or may not be stirred. The advantage of stirring is that it provides for better heat transfer. However, the disadvantage of stirring is that only a small amylose flock is formed, which is difficult to separate. In the absence of stirring, a larger amylose flock is formed which is properly separable. Moreover, in a non-stirred reaction mixture the transfer of oxygen is poor, which is advantageous with regard to the prevention of microbial decay. In general, a non-stirred reaction mixture is preferred.

In the practice of the method according to the invention, in the starch solution a solid amylose-rich phase is formed, which comprises more than 40 wt.% of amylose (based on the solids). This solid product can be separated from the solution, for instance by filtration, centrifugation or separation. The separated amylose product can subsequently be washed and dried. Amylose products have good properties as gelling agents and film formers. By virtue of these properties, amylose products can be used advantageously for various applications, as described in the above-mentioned publication by A.H. Young.

After the solid amylose-rich material has been separated from the reaction medium an amylopectin solution is left, which as such can be processed further into starch hydrolysates. However, the dissolved amylopectin can also be isolated from the solution through known methods, for instance through salting out with magnesium sulfate. It is also possible to dry the amylopectin solution. The amylopectin products obtained have good properties as thickener, binder and stabilizer. By virtue of these properties, amylopectin products can be used advantageously for various applications as described in the above-mentioned publication by A.H. Young.

The invention is further illustrated and explained in and by the following examples. In these examples a number of terms and assessment methods are used which are explained hereinafter. The 'yield' is defined as the total amount of solids of the separated amylose-rich fraction. 'Amylose-rich fraction' is understood to mean the separated starch products having an amylose content exceeding 40 wt.% based on the solids. The starting material potato starch has an amylose content of approximately 23 wt.%. The amylose content of the starch products is determined by means of gel permeation chromatography (GPC) and through assessment of the iodine blue value. The purity of the amylose rich fraction is understood to mean the amylose content in percent by weight, based on the solids. The purity of the enriched amylopectin fraction is understood to mean the amylopectin content in percent by weight based on the solids. The molecular weight of the amylose molecules present in the separated amylose fraction is determined by means of gel permeation chromatography (GPC). The degree of branching of the starch products is determined by first de-branching the starch molecules with isoamylase and subsequently determining the reducing power of the debranched starch hydrolysate.

### Example 1

a) Preparation of the enzyme composition
   3.4 kg potatoes (variety Bintje) were washed and chopped up in the presence of 0.1% (w/w) sodium bisulfite. The chopped potatoes were homogenized with a juice centrifuge and separated into two fractions, viz. potato juice on the one hand and starch + fibers on the other. The potato juice was subsequently centrifuged. The clear supernatant (designated as S1) was subjected to a heat treatment in a tubular reactor, which was maintained at a temperature of 54°C. The supernatant S1 was pumped through this tubular reactor with a residence time of 15 minutes. Subsequently the resultant protein precipitate was removed from the liquid by centrifugation. The residual clear liquid phase is designated as S2. The phosphorylase activity in S1 and S2 was 5.4 units/ml and 3.4 units/ml, respectively. The amylase activity in S1 and S2 was 1.623 units/ml and 0.014 units/ml, respectively.
b) Fractionation in the presence of inorganic phosphate without stirring
   3125 g potato starch (20 wt.% moisture) was added to water with stirring to form a 20-liter suspension. The starch suspension was gelatinized by means of a jet cooker at 120°C. The resultant starch solution was cooled to 37°C. Enzyme preparation was added with stirring (304 ml S2; 50 units phophorylase per liter reaction mixture). The concentration of water-soluble inorganic phosphate in the mixture was adjusted to 20 micromol per liter. The pH of the reaction mixture was adjusted to 7.0 with diluted hydrochloric acid. The reaction mixture was maintained at a temperature of 30°C for 20 h without stirring. Subsequently the reaction mixture was stored at 4°C for 24 h. The resultant solid amylose-rich fraction was separated from the liquid phase by means of centrifugation (Beckman, type J2-21M/E, Rotor JA-14) for 10 minutes at 5,000 rpm and room temperature. The amylose product was subsequently washed twice with demineralized water and centrifuged. The yield of dried amylose product was 333 g, corresponding with 13 wt.% of the potato starch (based on the solids). In the residual liquid the enriched amylopectin fraction is dissolved.
   The isolated amylose-rich fraction and the dissolved enriched amylopectin fraction were characterized by means of GPC, iodine colour and degree of branching. The amylose-rich fraction had a purity of 75%. The degree of branching of the amylose-rich fraction was 0.19 mol.%. The molecular weight of the amylose molecules was 340,000 Dalton (weight average). The enriched amylopectin fraction had a purity of 96%. The degree of branching of the amylopectin was 3.5 mol.%.

### Example 2

According to this example fractionation took place in the presence of water-soluble inorganic phosphate in a stirred reactor. 250 g potato starch (20 wt.% moisture) was added to water with stirring to form a 2-liter suspension. The starch suspension was gelatinized by means of a jet cooker at 120°C. The starch solution was cooled to 37°C. Subsequently 31 ml enzyme preparation S2 (obtained according to example la) was added with stirring. The concentration of inorganic phosphate was adjusted to 20 micromol per liter. The pH of the reaction mixture was adjusted to 7 with diluted hydrochloric acid. The reaction mixture was maintained at a temperature of 40°C for 24 h with stirring (12 rpm). Subsequently the reaction mixture was stored at 4°C for 24 h. The solid amylose-rich phase obtained was separated from the liquid phase by centrifugation (as in example 1b), washed and dried. The yield of amylose product was 26 g. The characterization of the two fractions was effected as decribed in example 1b. The amylose-rich fraction had a purity of 47%. The degree of branching of the amylose-rich fraction was 1.20 mol.%. The molecular weight of the amylose molecules was 280,000 Dalton (weight average). The enriched amylopectin fraction had a purity of 96%. The degree of branching of the amylopectin fraction was 3.7 mol.%. It appears from this example that stirring the reaction mixture has an adverse influence on the purity of the amylose-rich fraction.

### Example 3

a) Preparation of the purified potato phosphorylase.
   The preparation S2 mentioned under la served as starting material for the purification.
   The crude enzyme preparation was purified by means of (F)ast (P)rotein (L)iquid (C)hromatography over a high-load mono Q column (ion exchanger).
   Prior to chromatography, the solution was first dialysed against 20mM Tris/HCL, pH = 7.6, in order to remove low molecular components and to exchange salts against the starting buffer for the ion exchange chromatography.
   During the chromatography a stepped salt gradient was provided (0-1M NaC1). The major part of the protein was eluted at a salt concentration of 0 to 0.5 M NaCl. At a salt concentration between 0.5 and 0.6 M NaCl, the phosphorylase was eluted from the column. Of the phosphorylase applied, 90-100% was recovered in one peak. The total amount of protein in this peak represented 4% of the total protein applied. The phosphorylase was contained in 20 mM Tris/HCl pH = 7.6 + 0.53 M NaCl.
   The purity of the phosphorylase could be demonstrated by means of electrophoresis. Upon electrophoresis on gradient gels, after protein staining with Coomassie Blue, one band could be demonstrated having a molecular weight of 200,000 Dalton.
   The activity of the purified phosphorylase is 1.5 units/ml.
b) Fractionation in the presence of inorganic phosphate without stirring.
   250 g potato starch (20% moisture) was added to water with stirring to form a 2-liter suspension. The starch suspension was gelatinized by means of a jet cooker at 150°C. Of the starch solution obtained, 864 ml was cooled to 37°C. The pH of the solution was adjusted to 7.0 with diluted acid. Added with stirring were 136 ml purified phosphorylase and 20 micromol/liter water-soluble inorganic phosphate. The reaction mixture was maintained at a temperature of 37°C for 24 h without stirring. The resultant solid amylose-rich fraction was centrifuged off, washed and dried. The yield of amylose product was 12.9 g. The amylose fraction had a purity of 80%.

### Example 4 (comparative example)

If example 3b is repeated, but without addition of phosphorylase, no fractionation as described in example 3b occurs. This demonstrates that the action of phosphorylase is necessary to obtain a properly separable solid amylose-rich phase.

### Example 5

According to this example fractionation took place in the presence of glucose-1-phosphate without stirring. The potato phosphorylase preparation used was obtained according to the method described in example 3a.

250 g potato starch (20% moisture) was stirred with water to form a 2-liter suspension. The starch suspension was gelatinized by means of a jet cooker at 150°C. Of the starch solution obtained, 864 ml was cooled to 37°C. The pH of this solution was adjusted to 7.0 with diluted acid. Added with stirring were 136 ml purified potato phosphorylase and 10 mM/l disodium-α-D-glucose-1-phosphate. The reaction mixture was maintained at a temperature of 37°C for 24 h without stirring. The resultant solid amylose fraction was centrifuged off, washed and dried. The yield of amylose product was 28.5 g. This amylose fraction had a purity of 97%. The degree of branching was 0.53%. The molecular weight of the amylose molecules was 590,000 Dalton (weight average).

### Example 6 (comparative example)

In this example it is demonstrated that potato phosphorylase in active form is necessary to obtain a properly separable solid amylose fraction.

Potato juice S1 (of example 1a) was heated on a boiling water bath for 5 minutes. By this boiling process, the potato phosphorylase present in the potato juice is largely inactivated. After the boiling process the precipitate was removed from the solution by centrifugation. The clear supernatant was used according to the method below, instead of the potato phosphorylase preparation which was used in example 1b.

The method according to example 1b was repeated, but instead of 304 ml S2 (obtained according to example 1a) 304 ml of the above-mentioned clear supernatant (with inactivated phosphorylase) was used. After incubation for 20 h at 30°C and storage at 4°C for 24 h, a turbid solution was obtained. It proved to be impossible to separate a solid amylose-rich product.

## Claims

1. A method for fractionating starch, characterized in that potato phosphorylase is allowed to act on an aqueous starch solution which further contains dissolved phosphate, and the solid amylose-rich fraction obtained is separated from the residual liquid phase of the reaction mixture.

2. A method according to claim 1, characterized in that potato phosphorylase is used in the form of de-proteinized potato juice.

3. A method according to claim 1, characterized in that potato phosphorylase is used in the form of a preparation obtained from potato juice through purification.

4. A method according to claims 1-3, characterized in that, as dissolved phosphate, an inorganic phosphate compound is used.

5. A method according to claims 1-3, characterized in that, as dissolved phosphate, glucose-1-phosphate is used.

6. A method according to claims 1-5, characterized in that the starch concentration in the reaction mixture is between 3 and 40 wt.%.

7. A method according to claim 6, characterized in that the starch concentration in the reaction mixture is between 5 and 30 wt.%.

8. A method according to claims 1-7, characterized in that the pH of the reaction mixture is between 5 and 9.

9. A method according to claim 8, characterized in that the pH of the reaction mixture is between 6 and 8.

10. A method according to claims 1-9, characterized in that the reaction temperature is between 0 and 60°C.

11. A method according to claim 10, characterized in that the reaction temperature is between 20 and 45°C.

12. A method according to claims 1-11, characterized in that the reaction mixture is not stirred during the performance of the fractionation.

13. A method according to claims 1-12, characterized in that, as starch, potato starch is used.
